# EUROPÄISCHE PATENTSCHRIFT

(11) **EP 1 493 730 B1**
(45) Veröffentlichungstag und Bekanntmachung des Hinweises auf die Patenterteilung: **07.10.2009**
(21) Anmeldenummer: 04014228.3
(22) Anmeldetag: 17.06.2004
(51) Int. Cl.: C07C 201/16, C02F 1/26

(54) **Verfahren zur Aufarbeitung von Nebenkomponenten der Dinitroluolherstellung**
Process for working up side products of the dinitrotoluene production
Procédé de traitement des sous-produits de la production de dinitrotoluène

(30) Priorität: 30.06.2003 DE 10329303
(43) Veröffentlichungstag der Anmeldung: 05.01.2005
(73) Patentinhaber: Bayer MaterialScience AG, 51368 Leverkusen (DE)
(72) Erfinder: Münnig, Jürgen, 41564 Kaarst (DE); Wastian, Dietmar, 41542 Dormagen (DE); Lorenz, Wolfgang, Dr., 41540 Dormagen (DE); Keggenhoff, Berthold, Dr., 47839 Krefeld (DE)

(56) Entgegenhaltungen:
- EP-A- 1 132 347
- DE-C- 10 143 800
- US-A- 5 756 867

## Beschreibung

Gegenstand der Erfindung ist ein Verfahren zur Aufarbeitung der bei der Herstellung von Dinitrotoluol (DNT) durch Nitrierung von Toluol anfallenden organischen Nebenkomponenten. Diese Nebenkomponenten werden mit dem Prozesswasser vom Roh-DNT abgetrennt. Eine Behandlung dieser Organika ist erforderlich um das Prozessabwasser einer biologischen Aufarbeitung zuführen zu können.

Bei den üblichen Verfahren zur Herstellung von Dinitrotoluol (DNT) aus Toluol und einem Gemisch aus Schwefel- und Salpetersäure (Nitriersäure) fallen das bei der Schwefelsäureaufkonzentrierung abdestillierte saure Reaktionswasser sowie alkalisches und saures Waschwasser aus der Reinigung des DNT als Abwässer an. Dieses Prozessabwasser enthält neben Mono- und Dinitrotoluol Nebenprodukte der Nitrierung wie zum Beispiel Mono-, Di- und Trinitrokresole (im folgenden unter dem Begriff Nitrokresole zusammengefasst), Pikrinsäure und Nitrobenzoesäuren. Da aromatische Nitroverbindungen in biologischen Kläranlagen schwer abbaubar sind und bakterientoxische Eigenschaften aufweisen, wird die Entfernung dieser Stoffe aus dem Abwasser angestrebt.

Der bisherige Stand der Technik zur Behandlung organischer Nebenkomponenten bei der Nitrierung aromatischer Verbindungen stellt sich folgendermaßen dar:

US-A-6,506,948 behandelt die Aufarbeitung von Waschwasser aus der Reinigung von DNT, das aus Toluol und Nitriersäure hergestellt wurde. Dabei wird das DNT aus saurem und alkalischen Waschwasser zurückgewonnen, wobei die organischen Nebenkomponenten in der alkalischen Wasserphase verbleiben. Eine Aufarbeitung dieser Nebenkomponenten wird nicht beschrieben, es wird lediglich allgemein auf eine mögliche chemische (Oxidation) oder physikalische (Adsorption) Vorbehandlung vor Einleitung in eine biologische Kläranlage verwiesen.

Die Behandlung von Nitrokresolen, die vom Produktstrom mittels alkalischer DNT-Wäsche abgetrennt werden, kann gemäß EP-A1-0 962 446 durch oxidativen Abbau mit Salpetersäure bei erhöhten Temperaturen erfolgen. Dies erfordert gemäß EP-A1-0 962 446 jedoch eine zusätzliche, eigene Verfahrenstufe. Dabei werden Temperaturen bis 180°C erforderlich. Eine Nachbehandlung durch Adsorption an Aktivkohle oder eine entsprechende Aufarbeitung in einer biologischen Kläranlage bleibt erforderlich.

Zum Abbau von Nitroverbindungen im alkalischen Waschwasser der Nitrobenzolaufarbeitung wird in US-A-5,232,605 eine Behandlung dieses Abwassers mit Salpetersäure bei Temperaturen bis 290°C und Drücken bis 130 bar beschrieben. Nach diesem eigenen, zusätzlichen Verfahrensschritt kann das Abwasser einer biologischen Kläranlage zugeführt werden.

Im Rahmen der Nitrobenzolherstellung wird in US-A-4,230,567 ebenfalls ein Abbau von Nitrophenolen in einem eigenen, zusätzlichen Verfahrensschritt bei erhöhtem Druck und Temperatur beschrieben. Dabei wird nach alkalischer Wäsche des Nitrobenzols das Waschwasser Temperaturen von 150°C bis 500°C bei Drücken von 50 bis 350 bar unter Inertgasatmosphäre ausgesetzt.

In US-A-4,597,875 wird vorgeschlagen, nach alkalischer Wäsche des DNT, saurer Fällung der Nitrokresolkomponenten aus dem Waschwasser und deren mechanischen Abscheidung, die Nitrokresolverbindungen in einem geeigneten Verbrennungsprozess zu verbrennen. Auch hier ist jedoch für die Aufarbeitung der Nitrokresolkomponenten ein zusätzlicher Verfahrensschritt erforderlich.

Aufgabe der vorliegenden Erfindung ist daher, ein Verfahren zur Abtrennung und Behandlung unerwünschter Nebenkomponenten der Nitrierung zur Verfügung zu stellen, das einfach und wirtschaftlich ist. Überraschenderweise gelingt diese Aufgabe im Vergleich zu bisherigen Prozessen mit verfahrenstechnisch einfachen Schritten ohne den Einsatz zusätzlicher prozessfremder Verfahrensschritte und Einsatzstoffe.

Die Erfindung betrifft ein Verfahren zur Aufarbeitung von organischen Nebenkomponenten, die bei der einstufigen oder zweistufigen Nitrierung von Toluol zu Dinitrotoluol entstehen, und die im sauren und alkalischen Abwasser aus der Wäsche des Dinitrotoluols und im wässrigen Destillat aus der Schwefelsäureaufkonzentrierung zusammen mit geringen Mengen an Mononitrotoluol und Dinitrotoluol vorliegen, dadurch gekennzeichnet, dass
a) die sauren und alkalischen Abwässer aus der Wäsche und das wässrige Destillat aus der Schwefelsäureaufkonzentrierung vereinigt werden, so dass sich ein pH-Wert von unter 5 (gemessen bei 70°C) einstellt, und anschließend die entstehende wässrige und organische Phase durch Phasentrennung getrennt werden, und
b) die organische Phase aus Schritt a) in den Nitrierprozess zurückgeführt wird.

Im üblichen Nitrierverfahren aromatischer Kohlenwasserstoffe wird der Kohlenwasserstoff mit einem Gemisch aus Schwefelsäure und Salpetersäure (Nitriersäure) zur Reaktion gebracht. Im Fall der Nitrierung von Toluol zu Dinitrotoluol ist neben dem einstufigen Prozess (EP-A2-908 442) eine zweistufige Nitrierung das allgemein gängige Verfahren. Im zweistufigen Verfahren wird zunächst Toluol mit Salpetersäure und Schwefelsäure zum Mononitroluol (MNT) umgesetzt (Monostufe). Nach Trennung des entstandenen Reaktionsgemisches in MNT und eine Säurephase, was in statischen Abscheidern oder in dynamischen Abscheidem ausgeführt werden kann, wird das MNT erneut mit Salpetersäure und Schwefelsäure zum Dinitrotoluol (DNT) umgesetzt (Di-Stufe). Die Schwefelsäurephase aus der Monostufe wird aufkonzentriert. In der Di-Stufe wird aufkonzentrierte Schwefelsäure eingesetzt. Das Reaktionsgemisch der Di-Stufe wird in eine organische Phase, das Roh-DNT, und eine Säurephase getrennt, wobei die Säurephase als Einsatz-Schwefelsäure der Mono-Stufe dienen kann oder einer Aufkonzentrierung zugeführt wird. Die Trennung dieses Reaktionsgemisches der Di-Stufe kann ebenfalls in statischen oder dynamischen Abscheidern erfolgen.

In allen Herstellverfahren von DNT durch Toluolnitrierung mit Nitriersäure fallen zwei Stoffströme an, die einer weiteren Aufarbeitung zugeführt werden müssen, das Roh-DNT und die durch Reaktionswasser und den Wasseranteil der eingesetzten Salpetersäure verdünnte Schwefelsäure.

Das Roh-DNT besteht im Allgemeinen im wesentlichen aus dem gewünschten Reaktionsprodukt mit bis zu 1,5 Gew.-% Schwefelsäure sowie überschüssiger Salpetersäure von 0,5 Gew.-% bis 1,2 Gew.-% und Nebenkomponenten der Nitrierung in einer Konzentration von bis zu ca. 1 Gew.-%. Die Nebenkomponenten sind im wesentlichen Nitrokresole, Pikrinsäure und Nitrobenzoesäuren. Üblicherweise wird das Roh-DNT in zwei bis vier Waschstufen mit Wasser von Säuren und Nebenkomponenten befreit. Das in diesem Prozess zugeführte Waschwasser kann in mindestens einer Waschstufe eine Base enthalten, wobei üblicherweise Natronlauge oder Natriumkarbonat in Konzentrationen von 2 - 10 Gew.-% eingesetzt wird. Während durch die neutrale Wasserwäsche weitgehend Schwefelsäure und Salpetersäure aus dem Nitrierprodukt entfernt werden, überführt die alkalische Wäsche auch salzbildende organische Komponenten wie zum Beispiel Nitrokresole, Pikrinsäure und Nitrobenzoesäuren in die wässrige Phase.

Als Waschwasser kann, außer für die einstufige alkalische Wäsche sowie die letzte Wasserwäsche, jeweils Frischwasser oder im Gegenstrom geführtes Waschwasser einer Folgestufe eingesetzt werden. Das eingesetzte Waschwasser kann aber auch Frischwasser, demineralisiertes Wasser oder Wasser einer geeigneten Qualität aus einem Folgeprozess des beschriebenen Nitrierverfahrens sein.

Die eingesetzten Mengen an Waschwasser betragen bevorzugt 15 - 90 Gew.-Teile, besonders bevorzugt 50 - 65 Gew.-Teile Waschwasser pro 100 Gew.-Teile DNT.

In der neutralen Wasserwäsche entsteht je nach eingesetzter Waschwassermenge und Waschwasserführung ein saures Prozessabwasser mit bevorzugten Säuregehalten von 1,0 bis 3,0 Gew.-% Salpetersäure und 2,0 - 6,0 Gew.-% Schwefelsäure sowie einem DNT Gehalt von einigen 1000 ppm. Die Konzentration an organischen Nebenprodukten der Nitrierung liegt im genannten Prozessabwasser im Allgemeinen zwischen 300 und 900 ppm.

Im Abwasserstrom der alkalischen Wäsche sind im Allgemeinen 3,0 bis 7,0 Gew.-% organische Nebenprodukte der Nitrierung, im wesentlichen Nitrokresole, Pikrinsäure und Nitrobenzoesäuren in Form ihrer wasserlöslichen Salze enthalten. Daneben kann dieser Abwasserstrom auch noch mehrere 1000 ppm DNT sowie 2,0 - 4,0 Gew.-% Salpetersäure und ca. 0,6 - 1,2 Gew.-% Schwefelsäure in Form ihrer wasserlöslichen Salze enthalten. Der Abwasserstrom der alkalischen Wäsche hat einen pH-Wert >7,0, vorzugsweise >7,5 (gemessen bei 80°C).

Die Waschstufen werden in geeigneten Apparaten, vorzugsweise in Wasch- bzw. Extraktionskolonnen oder in Mischer-Scheider Apparaturen durchgeführt.

Die verdünnte Schwefelsäure aus der Nitrierung kann zu 70 - 90 Gew.-%, bevorzugt zu 70 - 80 Gew.-%, besonders bevorzugt zu 75 - 79 Gew.-%, aus Schwefelsäure bestehen und kann noch 0,005 bis 0,5 Gew.-%, vorzugsweise 0,005 bis 0,05 Gew.-% Salpetersäure sowie bis zu 3,0 Gew.-% MNT und 0,2 bis 2,0 Gew.-% DNT enthalten. Daneben enthält die aufzuarbeitende Säure noch bis zu 0,2 Gew.-% organische Nebenkomponenten, die im wesentlichen aus Nitrokresolen, Pikrinsäure und Nitrobenzoesäuren bestehen. Für die Aufkonzentrierung der verdünnten Schwefelsäure stehen neben anderen möglichen Verfahren zum Beispiel das Pauling-Verfahren bei Normaldruck [Bodenbrenner, von Plessen, Vollmüller, Dechema-Monogr. 86 (1980), 197] mit einer ca. 97 %igen Schwefelsäure als Produkt, sowie die Vakuumeindampfung [DE-A1-196 42 328], die ebenfalls eine Schwefelsäure bis 97 % liefern kann, zur Verfügung. Neben der gewünschten Schwefelsäure erhält man nach Kondensation der Brüden im Allgemeinen eine oder mehrere wässrige Phasen mit einem Schwefelsäureanteil von 0,2 bis 1,0 Gew.-%, vorzugsweise 0,2 - 0,6 Gew.%, und Gehalten an MNT von 0,7 bis 7,0 Gew.-% sowie DNT von 2,0 bis 6,0 Gew.-%. Weitere organische Verbindungen sind üblicherweise in Konzentrationen von bis zu 0,4 Gew.-% enthalten. Die organischen Anteile im Destillat sind gelöst bzw. dispergiert.

Im erfindungsgemäßen Verfahren werden die Abwasserströme der neutralen und der alkalischen DNT-Wäsche und der Schwefelsäureaufkonzentrierung vereint. Die wässrigen Phasen aus der Nitrierung setzen sich dabei aus mehreren, vorzugsweise zwei bis vier Einzelströmen zusammen, wobei mindestens einer der Einzelströme aus der neutralen Wasserwäsche stammt (saures Waschwasser) und mindestens ein Einzelstrom aus der alkalischen Wäsche stammt (alkalisches Waschwasser). Die Wasserphasen aus der Schwefelsäureaufkonzentrierung bestehen aus einem oder mehreren Einzelströmen mit genannten Anteilen an Säuren und organischen Komponenten

Die Vereinigung der Prozessabwasserströme kann in einem dafür geeigneten Behälter mit dynamischem Mischelement oder mittels einer statischen Mischeinheit erfolgen. Nach der Zusammenführung der genannten Ströme stellt sich ein pH-Wert der Mischung unterhalb von 5 (gemessen bei 70°C), vorzugsweise unterhalb von 2 ein, wobei sich eine organische Phase abscheidet. Wenn die alkalische Wäsche mit sehr großen Mengen an Base durchgeführt wird, dann ist es theoretisch möglich, dass bei der Vereinigung der Abwässer aus der DNT-Wäsche und des Destillats aus der Schwefelsäureaufkonzentrierung ein pH-Wert ≥ 5 erhalten wird. Dann müsste zum Beispiel die Menge des eingesetzten alkalischen Abwassers entsprechend reduziert werden, um einen pH-Wert von kleiner 5 zu erreichen. Diese organische Phase besteht aus MNT und DNT sowie Nebenprodukten der Nitrierung, vorwiegend Nitrokresolen, Pikrinsäure und Nitrobenzoesäuren. Im Falle der Benutzung von Karbonat in der alkalischen Wäsche muss eine entsprechende Entgasungsmöglichkeit beim Zusammenführen der Ströme vorhanden sein. Zur Abtrennung der gebildeten organischen Phase werden die vereinigten Abwasserströme anschließend in ein geeignetes Scheidegefäß geleitet. Die wässrige Phase wird nach dieser Abscheidung separat einer weiteren Abwasseraufarbeitung zugeführt.

Bei der Vereinigung der Prozessabwasserströme kann zusätzlich MNT eingespeist werden. Eine MNT-Zugabe kann die Phasentrennung unterstützen und durch Absenkung des Festpunktes der organischen Phase die Förderung dieses Stoffgemisches im Prozess erleichtern. Die eingesetzten Mengen betragen bevorzugt 0,2 - 9 Gew.-Teile, besonders bevorzugt 0,5 - 4,0 Gew.-Teile MNT pro 100 Gew.-Teile Prozessabwasser.

Aufgrund des Dichteunterschieds bildet die organische Phase, vorwiegend bestehend aus MNT, DNT, Nitrokresolen, Pikrinsäure und Nitrobenzoesäuren, im Allgemeinen die schwerere Phase. Im erfindungsgemäßen Verfahren wird diese organische Phase aus dem Scheidegefäß in den Nitrierprozess zurückgeführt. Dabei kann die Zurückführung der organischen Phase beim einstufigen Nitrierverfahren (wie zum Beispiel der adiabaten Dinitrierung) direkt in den Nitrierreaktor und beim zweistufigen Verfahren in die Nitrierreaktion zur MNT-Herstellung (Mono-Stufe) oder in die Nitrierreaktion zur DNT-Herstellung (Di-Stufe) erfolgen. Das mit den Abwässern ausgetragene MNT und DNT wird durch die Rückführung in den Nitrierprozess zurückgewonnen und die Nebenkomponenten wie Nitrokresole, Pikrinsäure und Nitrobenzoesäuren werden unter den oxidativen Bedingungen durch die im Nitrierprozess vorhandene Salpetersäure überraschenderweise oxidativ abgebaut. Dies zeigt sich zum Beispiel in der Entstehung von Oxalsäure als Abbauprodukt.

Der Vorteil des erfindungsgemäßen Verfahrens besteht somit darin, dass die im genannten Nitrierprozess gebildeten und über die DNT-Wäsche und anschließende Abwasservereinigung mit Organika-Abscheidung abgetrennten unerwünschten Nebenkomponenten auf einfache Weise ohne zusätzlichen Verfahrensschritt und ohne die Zufuhr zusätzlicher, prozessfremder Stoffe abgebaut werden können. Zusätzlich werden durch das genannte Verfahren über die Prozessabwässer ausgetragenes MNT und DNT zurückgewonnen.

### Beispiele:

### Beispiel 1:

500 g Nitriersäuregemisch, bestehend aus 79 Gew.-% Schwefelsäure, 10 Gew.-% Salpetersäure und 11 Gew.-% Wasser werden bei 70°C mit 10 g Gemisch aus der organischen Phase, erhalten nach Zusammenführung der Prozessabwässer aus DNT-Wäsche und Schwefelsäureaufkonzentrierung und nachfolgender Phasentrennung, versetzt.

Die eingesetzte organische Phase hat folgende Zusammensetzung:

| | |
|---|---|
| 13,2 % | MNT |
| 69,0 % | DNT |
| 17,0 % | Nitrokresole |
| 0,8 % | Pikrinsäure |

Über die Beobachtungszeit wird folgender Konzentrationsverlauf im Reaktionsgemisch ermittelt:

| Reaktionszeit [min.] | Σ Nitrokresole [ppm] | Pikrinsäure [ppm] |
|---|---|---|
| 0 | 3.342 | 149 |
| 1 | 1.764 | 144 |
| 5 | 895 | 143 |
| 15 | 672 | 141 |
| 30 | 537 | 134 |
| 60 | 398 | 132 |
| 120 | 255 | 127 |
| 360 | 115 | 115 |

In der Reaktionszeit erfolgt ein Abbau der Nitrokresolkonzentration um 96,6 Gew.-%. Die Pikrinsäurekonzentration nimmt um 22,8 Gew.-% ab.

## Patentansprüche

1. Verfahren zur Aufarbeitung von organischen Nebenkomponenten, die bei der einstufigen oder zweistufigen Nitrierung von Toluol zu Dinitrotoluol entstehen, und die im sauren und alkalischen Abwasser aus der Wäsche des Dinitrotoluols und im wässrigen Destillat aus der Schwefelsäureaufkonzentrierung zusammen mit geringen Mengen an Mononitrotoluol und Dinitrotoluol vorliegen, **dadurch gekennzeichnet, dass**
a) die sauren und alkalischen Abwässer aus der Wäsche und das wässrige Destillat aus der Schwefelsäureaufkonzentrierung vereinigt werden, so dass sich ein pH-Wert von unter 5 einstellt, und anschließend die entstehende wässrige und organische Phase durch Phasentrennung getrennt werden, und
b) die organische Phase aus Schritt a) in den Nitrierprozess zurückgeführt wird.

2. Verfahren nach Anspruch 1, bei dem die Vereinigung der wässrigen Abwässer in einem statischen Mischer mit nachgeschalteter Entgasung erfolgt.

3. Verfahren nach Anspruch 1 oder 2, bei dem die vereinigten wässrigen Phasen mit Mononitrotoluol vermischt werden.

4. Verfahren nach Anspruch 1 oder 2, bei dem die organischen Nebenkomponenten Nitrokresole enthalten.

5. Verfahren nach Anspruch 1 oder 2, bei dem die organischen Nebenkomponenten Pikrinsäure enthalten.

6. Verfahren nach Anspruch 1 oder 2, bei dem die organischen Nebenkomponenten Nitrobenzoesäuren enthalten.

## Claims

1. Process for working up organic secondary components which are formed in the one-stage or two-stage nitration of toluene to dinitrotoluene, and which are present in the acidic and alkaline waste water from the dinitrotoluene washing step and in the aqueous distillate from the sulfuric acid concentration step together with small amounts of mononitrotoluene and dinitrotoluene, **characterized in that**
a) the acidic and alkaline waste waters from the washing step and the aqueous distillate from the sulfuric acid concentration step are combined so that the resulting pH is below 5, and the aqueous and organic phases formed are then separated by phase separation, and
b) the organic phase from step a) is recycled into the nitration process.

2. Process according to Claim 1 wherein the aqueous waste waters are combined in a static mixer with a downstream venting facility.

3. Process according to Claim 1 or 2 wherein the combined aqueous phases are mixed with mononitrotoluene.

4. Process according to Claim 1 or 2 wherein the organic secondary components contain nitrocresols.

5. Process according to Claim 1 or 2 wherein the organic secondary components contain picric acid.

6. Process according to Claim 1 or 2 wherein the organic secondary components contain nitrobenzoic acids.

## Revendications

1. Procédé de traitement de sous-produits organiques se formant lors de la nitrification en une ou en deux étapes de toluène en dinitrotoluène, et qui sont présents dans les eaux résiduaires acides et alcalines provenant du lavage du dinitrotoluène et dans le distillat aqueux provenant de la concentration de l'acide sulfurique avec des quantités faibles de monodinitrotoluène et de dinitrotoluène, **caractérisé en ce que**
a) les eaux résiduaires acides et alcalines provenant du lavage et le distillat aqueux provenant de la concentration de l'acide sulfurique sont réunis de telle sorte qu'on obtient un pH inférieur à 5 et ensuite la phase aqueuse et la phase organique se formant sont séparées par séparation de phases et
b) la phase organique de l'étape a) est ramenée dans le processus de nitrification.

2. Procédé selon la revendication 1, dans lequel la réunion des eaux résiduaires aqueuses s'effectue dans un mélangeur statique avec dégazage ultérieur.

3. Procédé selon la revendication 1 ou 2, dans lequel les phases aqueuses réunies sont mélangées à du mononitrotoluène.

4. Procédé selon la revendication 1 ou 2, dans lequel les sous-produits organiques contiennent des nitrocrésols.

5. Procédé selon la revendication 1 ou 2, dans lequel les sous-produits organiques contiennent de l'acide picrique.

6. Procédé selon la revendication 1 ou 2, dans lequel les sous-produits organiques contiennent des acides nitrobenzoïques.
